Europäisches Patentamt

European Patent Office

Office européen des brevets

(19)

(11) Veröffentlichungsnummer: **0 013 763**
**A1**

(12)

# EUROPÄISCHE PATENTANMELDUNG

(21) Anmeldenummer: **79105406.7**

(22) Anmeldetag: **29.12.79**

(51) Int. Cl.³: **A 61 G 9/00**

(30) Priorität: **11.01.79 DE 2900806**

(43) Veröffentlichungstag der Anmeldung: **06.08.80**
**Patentblatt 80/16**

(84) Benannte Vertragsstaaten: **AT BE CH DE FR GB IT LU NL SE**

(71) Anmelder: **Intermedicat GmbH, Gerliswilstrasse 45, CH-6020 Emmenbrücke (CH)**

(72) Erfinder: **Heise, Peter, Heiligenbergstrasse 23, D-3501 Dörnhagen (DE)**
Erfinder: **Dinter, Jürgen, Quergasse 11, D-3508 Meisungen (DE)**

(74) Vertreter: **Selting, Günther, Dipl.-Ing. et al, Deichmannhaus am Hauptbahnhof, D-5000 Köln 1 (DE)**

(54) **Vorrichtung zum Sammeln einer Körperflüssigkeit, insbesondere Urin.**

(57) Die Vorrichtung zum Sammeln einer Körperflüssigkeit, insbesondere Urin, weist einen Sammelbeutel (3) auf, der über einen Zuleitungsschlauch (5) an einen Katheter angeschlossen wird. Zwischen dem Sammelbeutel (3) und dem Zuleitungsschlauch (5) befindet sich ein als Keimsperre dienendes Tropfgehäuse (2). Das Tropfgehäuse (2) ist fest mit einer Aufhängeplatte (1) verbunden, die am Krankenbett befestigt werden kann. Der Sammelbeutel (3) ist an der Aufhängeplatte (1) leicht ablösbar befestigt und kann ausgewechselt werden, ohne dass das Tropfgehäuse (2) oder der Zuleitungsschlauch (5) bewegt werden müssen.

ACTORUM AG

Vorrichtung zum Sammeln einer Körperflüssigkeit, insbesondere Urin

Die Erfindung betrifft eine Vorrichtung zum Sammeln einer Körperflüssigkeit, insbesondere Urin, mit einem Sammelbeutel, der über einen Zuleitungsschlauch an einen Katheter oder dergleichen angeschlossen ist, wobei in die Verbindung zwischen dem Sammelbeutel und dem Zuleitungsschlauch ein als Keimsperre dienendes Tropfgehäuse mit einem Belüftungsfilter zwischengeschaltet ist.

Um zu verhindern, daß bei gefülltem Sammelbeutel zum Anschluß eines neuen Beutels der Zuleitungsschlauch vom Katheter abgekuppelt werden muß, was für den Patienten schmerzhaft ist und sowohl für diesen als auch für die Krankenschwester die Gefahr einer Übertragungsinfektion mit sich bringt, ist gemäß DE-OS 22 39 180 vorgesehen, daß ein Urinsammelbehälter mit einem speziell gestalteten Ablaßhahn ausgerüstet wird, der sich mit einer Hand bedienen läßt und mit dem der Sammelbehälter entleert werden kann, ohne daß er vom Patienten abgetrennt werden muß. Der gesammelte Urin läuft durch den geöffneten Ablaßhahn in ein unter den Auslauf zu haltendes offenes Gefäß.

Ein Sammelbeutel nach DE-OS 25 45 295 ist zusätzlich zu einer Ablaßvorrichtung mit einem als Keimsperre dienenden Tropfgehäuse versehen, das ein Belüftungsfilter aufweist. Das Tropfgehäuse ist über ein gekrümmtes Einlaß-

0013763

verbindungsstück fest und unlösbar mit dem Sammelbeutel verbunden. An der Anschlußstelle zwischen Einlaßverbindungsstück und Sammelbeutel befindet sich ein Klappenventil, das das Strömen von Flüssigkeit aus dem Einlaßverbindungsstück in den Sammelbeutel zuläßt, jedoch das Strömen von Flüssigkeit aus dem Sammelbeutel in das Einlaßverbindungsstück verhindern soll.

Bei den beiden genannten Urinsammelbehältern ergibt sich eine unzumutbare Handhabung beim Entleeren sowie Geruchsbelästigung aller Anwesenden. Das offene Hantieren mit keimhaltigem Sammelurin beim Entleeren des gefüllten Sammelbeutels ist wegen der Keimverbreitung und Selbstkontamination gefährlich. In dem mehrfach wiedergefüllten Beutel verbleiben im übrigen stets Restkeime, die sich im erneuten Zulauf massiv vermehren, so daß eine Massen-Anzucht von Hospitalkeimen auftritt. In der Ablaßvorrichtung verbleibt Urin, der mit Sicherheit verkeimt. Das an den Sammelbeutel fest angebaute Tropfgehäuse ist als Keimsperre nicht unbedingt zuverlässig, weil durch seine Lage und bauartbedingte Kürze sowie die sich in der Krümmung des Einlaßverbindungsstückes und an der Ventilklappe ansammelnden Restkeime eine Kontamination des Tropfgehäuses eintreten kann, die zu einer Keimascension in der Harnableitung führen kann, so daß keine sichere Gewähr für die Verhinderung aufsteigender Harnweginfektionen gegeben ist.

Außerdem ist eine Urinableitung bekannt, bei der der Sammelbeutel abnehmbar an das Tropfgehäuse angeschlossen ist, so daß er bei erschöpfter Aufnahmekapazität von dem Tropfgehäuse gelöst und durch einen neuen Sammelbeutel ersetzt werden kann. Das Tropfgehäuse besteht bei dieser Anordnung aus einem flexiblen Schlauchabschnitt, der oben und unten mittels einer ebenen Platte verschlossen ist, und der eine Filtertüte enthält. In die Mitte der oberen Platte ist ein Tropfer eingesetzt, der mit einem zum Katheter führenden Schlauch verbunden ist. Die untere Platte trägt einen Anschlußstutzen für einen an den Urinbeutel angeschlossenen Schlauch. Die beiden Schläuche bilden mit dem zwischengeschalteten Tropfgehäuse eine insgesamt flexible, auf ihrer Länge nirgends stationär gehaltene Leitung zwischen Patient und Sammelbeutel. Dies hat zur Folge, daß der Patient immer unmittelbar mit dem Sammelbeutel in Verbindung steht, so daß er von jeder Verlagerung der das Tropfgehäuse enthaltenden Leitung belästigt und von jedem Wechsel des Sammelbeutels schmerzhaft beeinträchtigt wird. Im übrigen werden beim Beutelwechsel häufig Keime in die Urinableitung eingeschleppt, die durch Vermehrung und Wanderung über den Katheter in die ableitenden Harnwege gelangen. Hierdurch entstehen häufig gravierende und chronische Leiden, wie Cystitis oder Pyelonephritis, die zu aufwendiger und unter Umständen erfolgloser Behandlung zwingen. Außer der Gefahr der aufsteigenden Harnweginfektion durch Keimeinschleppung beim Beutelwechsel, kann das herkömmliche System Blasenschleimhautverletzungen durch eine Saugwirkung auf die den Katheteröffnungen anliegenden Schleimhautareale erzeugen, indem durch Auseinanderdrängen der Urinbeutelwände infolge des einfliessenden Urins ein auch an den Katheteröffnungen wirksamer Unterdruck entsteht.

Der Erfindung liegt die Aufgabe zugrunde, ein steriles, geschlossenes System für die Harnableitung so auszubilden, daß die Verbindung zwischen dem Katheter und dem Patienten so angebracht werden kann, daß der Patient von jeglichem Hantieren mit dem Sammelbeutel unbeeinträchtigt bleibt, so daß er von dem Wechsel des Sammelbeutels nicht gestört oder geschädigt wird. Ferner soll eine Keimsperre Keimascension zuverlässig verhindern und das Auftreten von Blasenschleimhautverletzungen durch Druckausgleich vermieden werden.

Dieser Aufgabenkomplex wird dadurch gelöst, daß das lösbar an den Sammelbeutel anschließbare Tropfgehäuse fest mit einer Aufhängeplatte verbunden ist, die Mittel zur abnehmbaren Anhängung des Sammelbeutels sowie zu ihrer eigenen waagerechten und lotrechten Montage aufweist.

Hierdurch läßt sich die gesamte Vorrichtung am Bett des an die Vorrichtung angeschlossenen Patienten so befestigen, daß die für die Keimsperrenfunktion erforderliche, ausreichend genau waagerechte und lotrechte Anbringung gewährleistet ist. Außerdem erfolgt das Auswechseln eines gefüllten Sammelbeutels gegen einen neuen Sammelbeutel hinter der in bezug auf den Patienten festgelegten Verbindung zwischen Katheter-Zuleitungsschlauch und Tropfgehäuse, so daß der Patient von diesem Vorgang praktisch unbeeinträchtigt bleibt. Das Tropfgehäuse verbleibt während der gesamten Liegedauer des Katheters an diesem. Auch hierdurch ergibt sich eine Schonung des Patienten. Der Sammelbeutel läßt sich einfach und schnell von der Aufhängeplatte abnehmen bzw. an diese anhängen, und es ist immer ein korrekter Anschluß zwischen Tropfgehäuse und Sammelbeutel gewährleistet. Da stets ein neuer Sammelbeutel mit dem Tropfgehäuse verbunden wird, ist die Vermehrung von Restkeimen durch in den Sammelbeutel nachlaufenden Urin aus-

0013763

geschlossen. Da das Tropfgehäuse mit der Aufhängeplatte unabhängig von dem Sammelbeutel stationär montiert ist, läßt es sich ausreichend groß bemessen und genau waagerecht und lotrecht so ausrichten, daß keine Kontamination der Tropfkammer auftritt und Keimascension in das Urinableitungssystem über der Tropfkammer wirkungsvoll vermieden wird.

Zur Benutzung der Vorrichtung wird die Aufhängeplatte mit dem Tropfgehäuse waagerecht und lotrecht an einem Bettrahmen befestigt und an diesem für die Liegedauer des Katheters belassen. Sodann wird ein Einmal-Urinsammelbeutel an der Aufhängeplatte lösbar befestigt, und es werden der mit dem Katheter in Verbindung stehende Zuleitungsschlauch sowie der Einlaß des Sammelbeutels an das Tropfgehäuse angeschlossen.Die waagerechte Befestigung der Aufhängeplatte an dem Bettrahmen bringt das Tropfgehäuse in seine senkrechte Normalstellung, in der der Urin durch die Tropfkammer des Tropfgehäuses pfützenfrei in den Sammelbeutel befördert wird.Ein Rückspritzen ablaufenden Urins wird vermieden, so daß Keimascension sicher verhindert wird. Die Einheit von Aufhängeplatte und Tropfgehäuse läßt sich preiswert herstellen und ist zum Ein-Patienten-Gebrauch bestimmt.

Vorteilhaft ist die Aufhängeplatte im wesentlichen eben ausgebildet und mit zwei Haken zur Anhängung des Sammelbeutels versehen. Sie kann als U-förmiger starrer Bügel gestaltet sein, an dessen oberem Steg das Tropfgehäuse befestigt ist und an dessen Schenkeln die Haken zur Anhängung des Sammelbeutels vorgesehen sind. Zur Befestigung der Aufhängeplatte an einem Bettrahmen ist sie mit Lochdurchbrechungen oder Zapfen zu ihrer waagerecht hängenden Befestigung versehen.

Das Tropfgehäuse ist vorteilhaft als sich nach unten trichterartig verjüngender flacher Kastenkörper ausgebildet, der an seinem tiefsten Teil einen Anschlußstutzen zur Verbindung mit dem Sammelbeutel aufweist, und in dessen Oberteil das an den Zuleitungsschlauch angeschlossene Tropfende so eingesetzt ist, daß sich das Tropfende senkrecht über einer Trichterschräge befindet. Es kann aus durchsichtigem, vorzugsweise glasklarem, starrem oder halbstarrem Material hergestellt sein. Bei waagerecht montierter Aufhängeplatte ist das Tropfgehäuse im wesentlichen senkrecht ausgerichtet. Die Form des Tropfgehäuse-Unterteils und die Lage des Tropfendes relativ zur Trichterschräge verhindern ein Rückspritzen ablaufenden Urins zum Tropfende, weil Einfallwinkel gleich Ausfallwinkel ist. Der freie Tropfenfall gewährleistet eine sichere Keimbarriere zur Verhinderung von Keimascension und ihren Folgen.

In einer Wand des Tropfgehäuses ist zweckmäßig eine von außen mit Filtermaterial abgedeckte Öffnung ausgebildet. Die Tropfgehäuseöffnung kann außen von Randprofilierungen zur Bildung einer umlaufenden Stecknut umgeben sein, in die ein entsprechender Flansch einer durchbrochenen Kappe passend eingreift. Das Filtermaterial ist zwischen den Randprofilierungen der Tropfgehäuseöffnung und der Kappe eingeklemmt gehalten. Hierdurch wird eine integrierte bakteriendichte Belüftung des Tropfgehäuses erreicht, die einen Druckausgleich zur Vermeidung von Blasenschleimhautverletzungen aufgrund von bei sich füllendem Sammelbeutel entstehendem Unterdruck vermittelt.

Als Filtermaterial können einfach ebene Filterscheiben verwendet werden, die für eine bakteriendichte Be- lüftung sorgen. Die Befestigung der Filterscheibe an der Öffnung durch Aufstecken der Kappe, die gegebenenfalls verleimt werden kann, ist einfach und trägt zu der Preiswürdigkeit der Vorrichtung bei, die ihrem Einsatz zum Ein-Patienten-Gebrauch angemessen ist.

In der Zeichnung ist ein Ausführungsbeispiel der Erfindung schematisch dargestellt. Es zeigt:

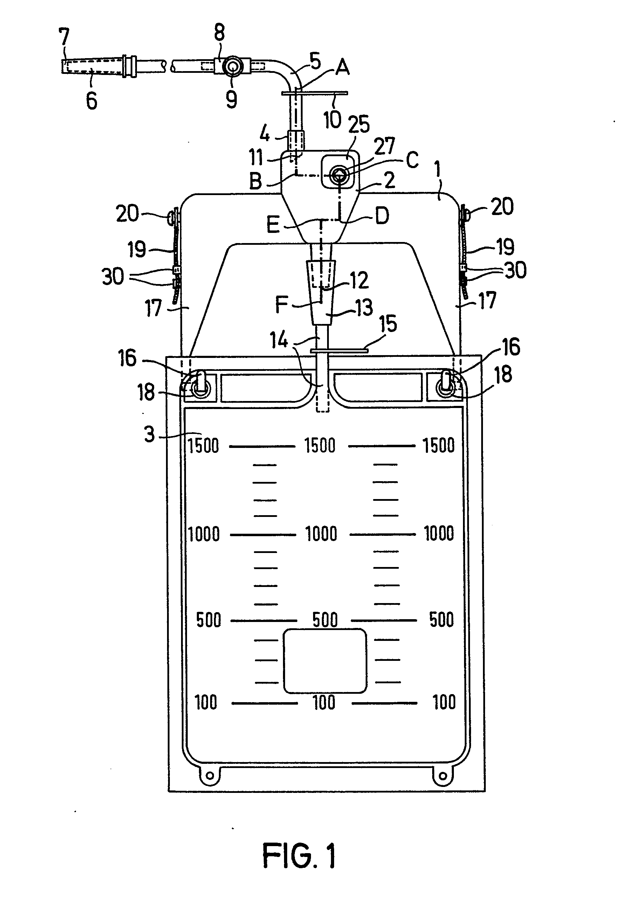

Fig. 1 eine Gesamtansicht der Vorrichtung mit Tropfgehäuse und Sammelbeutel,

Fig. 2 einen Längsschnitt des Tropfgehäuses (A-F in Fig. 1), sowie der abgenommen dargestellten Kappe zum Verschluß der Belüftungsöffnung,

Fig. 3 eine Draufsicht der Kappe für die Belüftungsöffnung.

Die erfindungsgemäße Vorrichtung besteht im wesentlichen aus einer ebenen Aufhängeplatte 1, mit der ein Tropfgehäuse 2 unlösbar verbunden ist, während ein Urinsammelbeutel 3 abnehmbar angehängt ist.

Das Tropfgehäuse 2 aus glasklarem Material trägt an der Seite seiner oberen Wand einen Stutzen 4 zur Montage eines Zuleitungsschlauches 5, der mittels eines Konusansatzes 6 an einen Harnröhrenkatheter angeschlossen wird, nachdem eine Schutzkappe 7 abgenommen worden ist. Zur Entnahme einer sterilen Probe Zwischenuntersuchungsurins ist der Zuleitungsschlauch 5 mit einem Zwischenstück 8 versehen, das eine Punktionsstelle 9 in Form einer gummielastischen Membran oder dergleichen enthält. Für die Probeentnahme wird die Membran mit einer Kanüle punktiert und die Probe in eine Spritze gesaugt. Beim Herausziehen der Kanüle aus der Membran verschließt sich die Einstichstelle infolge der inneren Spannung der Membran automatisch wieder. Um eine gewisse Ansammlung von Probeurin in dem Zuleitungsschlauch 5 zu erreichen, wird eine Schlauchschiebeklemme 10 betätigt. Das Ende des Zuleitungsschlauches 5 ist zur Bildung eines Tropfers 11 abgeschrägt und ragt durch den Stutzen 4 hindurch in den oberen Bereich des Tropfgehäuses 2.

Das Tropfgehäuse 2 ist kastenförmig mit einer unteren trichterartigen Verlängerung gestaltet. An der tiefsten Stelle des Unterteils befindet sich ein konischer Anschlußstutzen 12, auf den ein entsprechendes Paßstück 13 eines in den Sammelbeutel 3 führenden Schlauches 14 abnehmbar aufschiebbar ist. Der Schlauch 14 kann mittels einer Schlauchschiebeklemme 15 kurzzeitig abgesperrt werden. Der Tropfer 11 befindet sich senkrecht über der Schrägfläche der trichterartigen Verlängerung, wodurch ein Rückspritzen von Flüssigkeit nach oben verhindert wird.

Zur hängenden abnehmbaren Befestigung des Sammelbeutels 3 an der Aufhängeplatte 1 dienen Haken 16, die an den unteren Enden von Schenkeln 17 ausgebildet sind, die durch die Gestaltung der ebenen Aufhängeplatte 1 als U-förmiger Bügel entstehen. Über die Haken 16 werden Ösen 18 am Sammelbeutel gehängt. Zum Anhängen der Aufhängeplatte 1 an einem Bettholm oder dergleichen dienen Zapfen 20, die nach entgegengesetzten Seiten vom oberen Steg abstehen. Zur Befestigung wird auf die Zapfen 20 das Loch eines Bandes 19 gezogen, und das andere Ende des Bandes 19 wird zwischen den Doppelstegen 30 an den Schenkeln 17 der Aufhängeplatte 1 eingeklemmt. Auf diese

0013763

Weise kann die Aufhängeplatte 1 mit dem Tropfgehäuse 2 waagerecht an einem Bettrahmen befestigt werden, so daß das Tropfgehäuse senkrecht an dem Bettrahmen montiert und zur Erzielung einer sicheren Keimbarriere ein freier Tropfenfall und ein pfützenfreier Übergang des Urins in den Sammelbeutel gewährleistet ist.

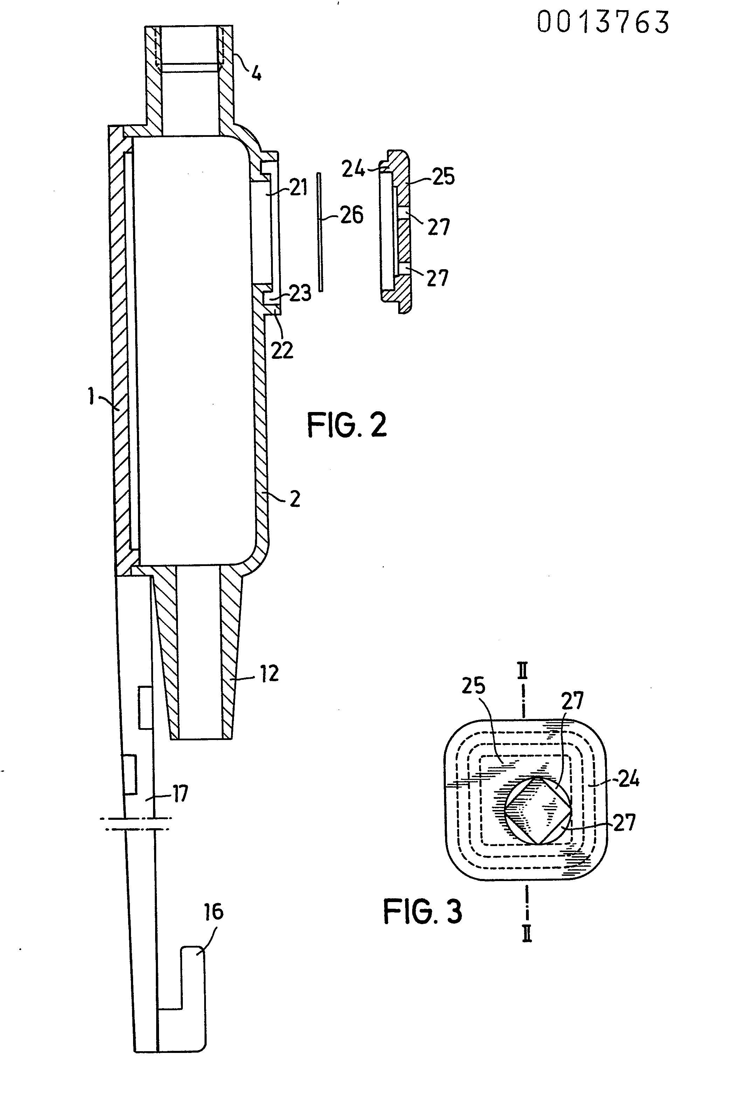

In der Vorderwand des Tropfengehäuses 2 befindet sich eine Öffnung 21 (Fig. 2), die außen von einer Randprofilierung 22 umgeben ist. Die Randprofilierung besteht aus zwei zueinander parallelen Rippen, die zwischen sich eine Stecknut 23 bilden. In die Stecknut 23 greift ein Flansch 24 einer Kappe 25 passend ein, wobei zwischen der inneren Randprofilierung 22 und der Kappe 25 eine bakteriendichte Filterscheibe 26 eingeklemmt wird. In der Kappe sind Belüftungsöffnungen 27 ausgebildet, die einen Druckausgleich zur Vermeidung des Auftretens von Unterdruck an der Katheteröffnung bewirken.

Nachdem die Aufhängeplatte 1 mittels der Bänder 19 an einem Bettrahmen befestigt worden ist, wird ein neuer Einmal-Sammelbeutel 3 an die Haken 16 der Aufhängeplatte 1 angehängt und der Anschluß zwischen dem Anschlußstutzen 12 und dem Paßstück 13 hergestellt. Sodann wird überden Zuleitungsschlauch 5 das Tropfgehäuse 2 mit einem Katheter verbunden. Aus dem Tropfende 11 tropft Urin in den Unterteil des Tropfgehäuses 2, wobei dessen verjüngte Form ein Rückspritzen in den Zueltiungsschlauch und damit Keimascension verhindert. Aus dem unteren Teil des Tropfgehäuses 2 gelangt der Urin durch den Anschluß 12,13 in den Sammelbeutel. WEnn dieser gefüllt ist, wird der Anschluß 12, 13 abgekuppelt, der Sammelbeutel wird von der Aufhängeplatte 1 abgehängt und durch einen neuen ersetzt. Der Beutelwechsel erfolgt unterhalb der Keimschranke vollkommen hygienisch, so daß keine Infektionsgefahr besteht.

Die gesamte aus Aufhängeplatte 1, Tropfgehäuse 2, Zuleitungsschlauch 5 und Sammelbeutel 3 bestehende Vorrichtung ist zum Ein-Patienten-Gebrauch bestimmt.

0013763

A N S P R Ü C H E

1. Vorrichtung zum Sammeln einer Körperflüssigkeit, insbesondere Urin, mit einem Sammelbeutel, der über einen Zuleitungsschlauch an einen Katheter oder dergleichen angeschlossen ist, wobei in die Verbindung zwischen dem Sammelbeutel und dem Zuleitungsschlauch ein als Keimsperre dienendes Tropfgehäuse mit einem Belüftungsfilter zwischengeschaltet ist, d a d u r c h   g e k e n n - z e i c h n e t ,   daß das lösbar an den Sammelbeutel (3) anschließbare Tropfgehäuse (2) fest mit einer Aufhängeplatte (1) verbunden ist, die Mittel (16;20) zur abnehmbaren Anhängung des Sammelbeutels (3) sowie zu ihrer eigenen waagerechten und lotrechten Montage aufweist.

2. Vorrichtung nach Anspruch 1,   d a d u r c h g e k e n n z e i c h n e t ,   daß die Aufhängeplatte (1) im wesentlichen eben ausgebildet und mit zwei Haken (16) zur Anhängung des Sammelbeutels (3) versehen ist.

3. Vorrichtung nach den Ansprüchen 1 oder 2, d a d u r c h
g e k e n n z e i c h n e t, daß die Aufhängeplatte (1)
als U-förmiger starrer Bügel gestaltet ist, an dessen
oberem Steg das Tropfgehäuse (2) befestigt ist und an
dessen Schenkeln (17) die Haken (16) zur Anhängung des
Sammelbeutels (3) vorgesehen sind.

4. Vorrichtung nach den Ansprüchen 1 bis 3, d a -
d u r c h g e k e n n z e i c h n e t, daß das
Mittel zur Montage der Aufhängeplatte (1) aus Lochdurchbrechungen im oberen Steg des U-förmigen Bügels
besteht.

5. Vorrichtung nach den Ansprüchen 1 bis 3, d a -
d u r c h g e k e n n z e i c h n e t, daß das
Mittel zur Montage der Aufhängeplattë (1) aus von
dem Steg des U-förmigen Bügels nach entgegengesetzten
Seiten ausgehenden Zapfen (20) besteht.

6. Vorrichtung nach den Ansprüchen 1 bis 5, d a -
d u r c h g e k e n n z e i c h n e t, daß das
Tropfgehäuse (2) als sich nach unten trichterartig
verjüngender flacher Kastenkörper ausgebildet ist,
der an seinem tiefsten Teil einen Anschlußstutzen
(12) zur Verbindung mit dem Sammelbeutel (3) aufweist
und in dessen Oberteil das an den Zuleitungsschlauch
(5) angeschlossene Tropfende (11) so eingesetzt ist,
daß sich das Tropfende (11) senkrecht über einer
Trichterschräge befindet.

7. Vorrichtung nach Anspruch 6, d a d u r c h
g e k e n n z e i c h n e t, daß das Tropfgehäuse
(2) aus durchsichtigem, vorzugsweise glasklarem,
starrem oder halbstarrem Material hergestellt ist.

0013763

8. Vorrichtung nach einem der Ansprüche 1 bis 7, d a - d u r c h   g e k e n n z e i c h n e t,   daß in einer Wand des Tropfgehäuses (2) eine von außen mit Filtermaterial (26) abgedeckte Öffnung (21) ausgebildet ist.

9. Vorrichtung nach Anspruch 8, d a d u r c h g e k e n n z e i c h n e t,   daß die Tropfgehäuse-Öffnung (21) außen von Randprofilierungen (22) zur Bildung einer umlaufenden Stecknut (23) umgeben ist, in die ein entsprechender Flansch (24) einer durchbrochenen Kappe (25) passend eingreift, und daß das Filtermaterial (26) zwischen den Randprofilierungen (22) und der Kappe (25) eingeklemmt gehalten ist.

FIG.1

0013763

FIG. 2

FIG. 3

**EUROPÄISCHER RECHERCHENBERICHT**

0013763

Nummer der Anmeldung

EP 79 10 5400

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 3 371 897 (F. SERANY et al.)<br><br>* Figuren 1-4; Spalte 1, Zeile 69 bis Spalte 2, Zeile 6; Spalte 2, Zeilen 49-65; Spalte 3, Zeilen 17-71 * | 1 |
| A | US - A - 3 534 738 (CH. HUCK)<br><br>* Figuren 1,8; Spalte 5, Zeilen 12-30 * | 1,7 |
| A | US - A - 3 716 055 (W. SCHULTZE)<br><br>* Figuren 1,2,7; Spalte 2, Zeilen 3-36 * | 1,3,5 |
| A | US - A - 3 661 153 (E. POLK et al)<br><br>* Figur 1; Spalte 1, Zeile 64 bis Spalte 2, Zeile 6 * | 1 |
| A | US - A - 3 800 795 (C. WALKER)<br><br>* Figuren 1-3,5; Spalte 2, Zeile 17 bis Spalte 3, Zeile 16 * | 1,6,8 |

**KLASSIFIKATION DER ANMELDUNG (Int.Cl. 3)**

A 61 G 9/00

**RECHERCHIERTE SACHGEBIETE (Int. Cl. 3)**

A 61 G
A 61 M
A 61 F

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 24-04-1980 | VEREECKE |

EPA form 1503.1   06.78